# EUROPEAN PATENT APPLICATION

(11) **EP 4 218 557 A1**
(43) Date of publication of application: **02.08.2023**
(21) Application number: 21881680.9
(22) Date of filing: 20.08.2021
(51) Int. Cl.: A61B 5/0205, A44C 5/18

(54) **ELECTRONIC DEVICE AND WEARABLE DEVICE**

(30) Priority: 23.10.2020 CN 202011148777
(71) Applicant: GUANGDONG OPPO MOBILE TELECOMMUNICATIONS CORP., LTD., Dongguan, Guangdong 523860 (CN)
(72) Inventor: ZHOU, Shun, Dongguan, Guangdong 523860 (CN)
(74) Representative: Novagraaf Technologies
(86) International application number: PCT/CN2021/113803
(87) International publication number: WO 2022/083261

(57) **Abstract**

The present application relates to the technical field of smart devices. Disclosed are an electronic device and a wearable device. In the electronic device, a contact surface is provided on an outer surface of a housing; an accommodating space is formed inside the housing; a first connector is provided on the housing and is used for electrical connection to a power source to transmit electric energy for the electronic device; first and second electrodes are provided on the contact surface at intervals and are used as measuring electrodes of an ECG signal detection circuit; each of the first and second electrodes is electrically connected to the first connector; and a main board is electrically connected to the first connector and is used for obtaining ECG parameters according to ECG signals acquired by the first and second electrodes. In the present application, the first and second electrodes for the ECG signal detection circuit are provided on the housing, and the first and second electrodes are connected to the first connector that provides electrical energy to achieve the sharing of the first connector, such that the cost is reduced, and in addition, the contact area between the electrodes and a human body is not limited by the housing itself, and the space occupied by the stacking of internal electronic components can be reduced, thereby miniaturizing the electronic device.

## Description

### TECHNICAL FIELD

The present disclosure relates to the field of smart devices, and in particular, to an electronic device and a wearable device.

### BACKGROUND

In order to realize the electrocardiogram (ECG) function, electrode pads need to be provided in a wearable device, further requiring grooves and metal inserts to be arranged on the housing of the wearable device. Therefore, the cost is relatively high, and even the stacking of related electronic components will occupy a large amount of inner space of the wearable device, resulting in a too bulky wearable device.

### SUMMARY

The present disclosure provides an electronic device and a wearable device.

In order to solve the above technical problem, a technical solution adopted in the present disclosure is an electronic device. The electronic device includes a housing, a first connecting head, a first electrode, a second electrode, and a mainboard. The housing has a contact surface on the outer surface thereof and an accommodating space inside. The first connecting head is arranged on the housing and is configured to be electrically connected with a power source to transmit electric energy for the electronic device. The first electrode and the second electrode are arranged on the contact surface apart from each other, and are configured as measuring electrodes of an electrocardiogram (ECG) signal detection circuit. Each of the first electrode and the second electrode is electrically connected to the first connector. The mainboard is electrically connected to the first connector, and is configured to obtain ECG parameters according to the ECG signals collected by the first electrode and the second electrode.

In order to solve the above technical problem, another technical solution adopted by the present disclosure is a wearable device. The wearable device includes a strap and the above-mentioned electronic device. The strap is connected to the housing, and the strap is configured to be able to wear the electronic device on a user's body.

In the above solutions, the present disclosure discloses that the first electrode and the second electrode configured to form the ECG signal detection circuit are arranged on the housing, and the sharing of the first connector that provides electric energy is realized through connecting both of the first electrode and the second electrode with the first connector. Therefore, the cost is reduced, and the contact area between the electrodes and the human body is not limited by the housing itself, and it may even save the space occupied by the stacking of internal electronic components, thus miniaturizing the electronic device.

### BRIEF DESCRIPTION OF THE DRAWINGS

FIG. 1 is a schematic structural view of a wearable device according to an embodiment of the present disclosure.
FIG. 2 is a schematic structural view of a wearable device according to another embodiment of the present disclosure.
FIG. 3 is a front view of the electronic device according to the embodiment shown in FIG. 1 of the present disclosure.
FIG. 4 is a rear view of the electronic device according to the embodiment shown in FIG. 1 of the present disclosure.
FIG. 5 is a partial schematic structural view of the electronic device according to the embodiment shown in FIG. 1 of the present disclosure.
FIG. 6 is a schematic structural view of the first housing according to the embodiment shown in FIG. 3 of the present disclosure.
FIG. 7 is a schematic structural view of the first housing according to another embodiment shown in FIG. 6 of the present disclosure.
FIG. 8 is an exploded schematic view of one perspective of the detachable connector according to the embodiment shown in FIG. 7 of the present disclosure.
FIG. 9 is an exploded schematic view of another perspective of the detachable connector according to the embodiment shown in FIG. 7 of the present disclosure.
FIG. 10 is a partial cross-sectional view of the housing and the connecting ring connected with each other according to the embodiment shown in FIG. 9 of the present disclosure.
FIG. 11 is a sectional view of a part C of the housing body and the connecting ring connected with each other according to the embodiment shown in FIG. 10 of the present disclosure.
FIG. 12 is a schematic structural view of the electronic device when being worn according to the embodiment shown in FIG. 7 of the present disclosure.
FIG. 13 is another schematic structural view of the electronic device when being worn according to the embodiment shown in FIG. 7 of the present disclosure.
FIG. 14 is another schematic structural view of the electronic device when being worn according to the embodiment shown in FIG. 12 of the present disclosure.
FIG. 15 is a schematic structural view of the second housing according to the embodiment shown in FIG. 4 of the present disclosure.
FIG. 16 is a partial schematic structural view of the electronic device according to the embodiment shown in FIG. 4 of the present disclosure.
FIG. 17 is a partial schematic structural view of the electronic device according to the embodiment shown in FIG. 4 of the present disclosure.
FIG. 18 is a schematic structural view of the mainboard assembly according to the embodiment shown in FIG. 5 of the present disclosure.
FIG. 19 is an exploded schematic view of the mainboard assembly according to the embodiment shown in FIG. 18 of the present disclosure.
FIG. 20 is a schematic structural view of a wearable device according to another embodiment of the present disclosure.

### DETAILED DESCRIPTION

The present disclosure will be further clearly and completely described below in conjunction with the accompanying drawings and embodiments. In particular, the following embodiments are only used to illustrate the present disclosure, but not to limit the scope of the present disclosure. Likewise, the following embodiments are only some but not all of the embodiments of the present disclosure, and all other embodiments obtained by person of ordinary skill in the art without creative efforts fall within the protection scope of the present disclosure.

Reference herein to an "embodiment" means that a particular feature, structure, or characteristic described in conjunction with the embodiment may be included in at least one embodiment of the present disclosure. The occurrences of this phrase in various places in the specification are not necessarily all referring to the same embodiment, nor are separate or alternative embodiments mutually exclusive of other embodiments. It is understood explicitly and implicitly by those skilled in the art that the embodiments described herein may be combined with other embodiments.

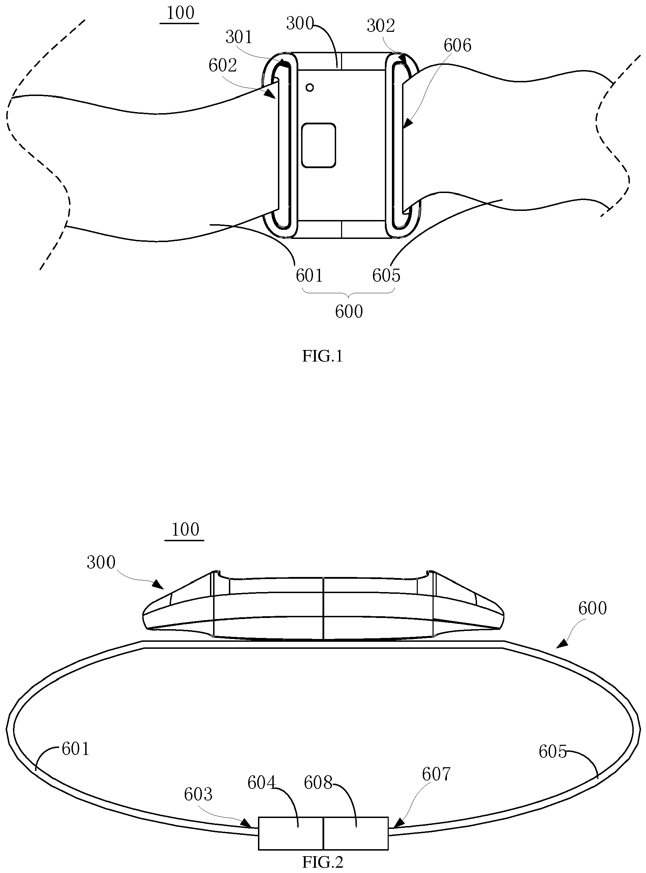

As shown in FIG. 1, which is a schematic structural view of a wearable device according to an embodiment of the present disclosure. The wearable device 100 may include an electronic device 300 and a strap 600. In the wearable device 100, the electronic device 300 may be worn on a user's body such as the wrist or the head through the strap 600. It can be understood that the strap 600 may be omitted.

As used herein, an "electronic device" (also may be referred to as a "terminal" or "mobile terminal" or "electronic device") includes but is not limited to a device for receiving/transmitting communication signals via a wired line or wireless interface. For example, the wired line may include Public Switched Telephone Network (PSTN), Digital Subscriber Line (DSL), digital cable, direct cable, and/or a data connection/network. For example, the wireless interface may include cellular networks, wireless local area networks (WLAN), digital TV network based on DVB-H network, satellite network, AM-FM broadcast transmitter, and/or another communication terminal. The communication terminal configured to communicate via a wireless interface may be referred to as a "wireless communication terminal", "wireless terminal" or "mobile terminal". Examples of mobile terminals include, but are not limited to, a satellite or a cellular telephone; a Personal Communications Systems (PCS) terminal that may combine cellular radiotelephones with data processing, facsimile, and data communication capabilities. Mobile terminals also may include a radiotelephone, a pager, an Internet/Intranet access, a web browser, an organizer, a calendar, and/or a PDA of a Global Positioning System (GPS) receiver; and a conventional laptop and/or a palm-type receiver or other electronic devices including a radiotelephone transceiver.

As shown in FIG. 1, the electronic device 300 is provided with a first end 301 and a second end 302. The first end 301 of the electronic device 300 is connected to one end of the strap 600. The second end 302 of the electronic device 300 is connected to one end of the strap 600. Thus, a ring structure is formed that may be worn by a user, which is convenient for the wearing of the wearable device 100.

The electronic device 300 may be the main body of a wearable measuring device such as a smart bracelet, a sports watch, a heart rate watch, a heart rate bracelet, an ECG watch, etc., or the main body of a wearable device such as a phone watch, a head-mounted display (the head-mounted display may send optical signals to the eyes through various head-mounted displays to achieve different effects such as virtual reality, augmented reality, and mixed reality), and a light. In other words, the electronic device 300 is the core part of the wearable device 100, so the electronic device 300 may also be called a "main body". The electronic device 300 considered as the main body of the ECG watch is taken as an example in the following description.

As shown in FIG. 2, which is a schematic structural view of a wearable device 100 according to another embodiment of the present disclosure. In the embodiment, the electronic device 300 may be directly arranged on the ring-structured strap 600, such that the user may directly wear the wearable device 100.

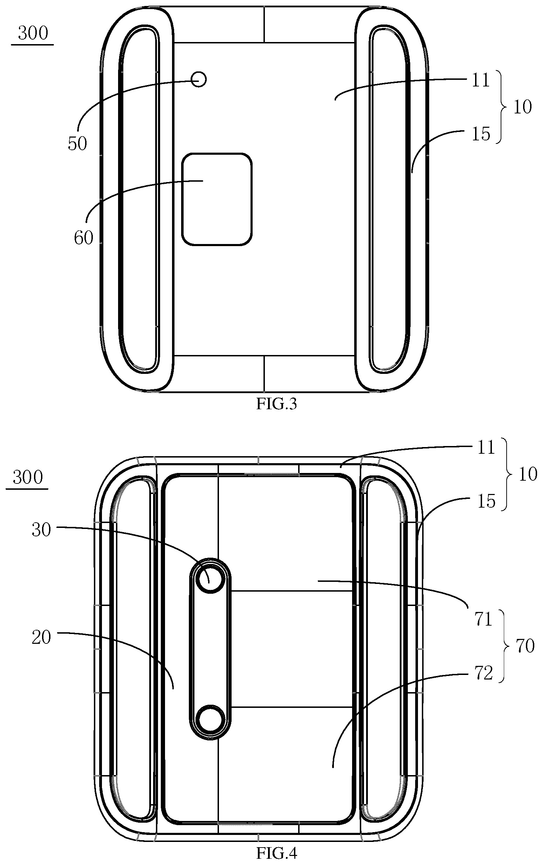

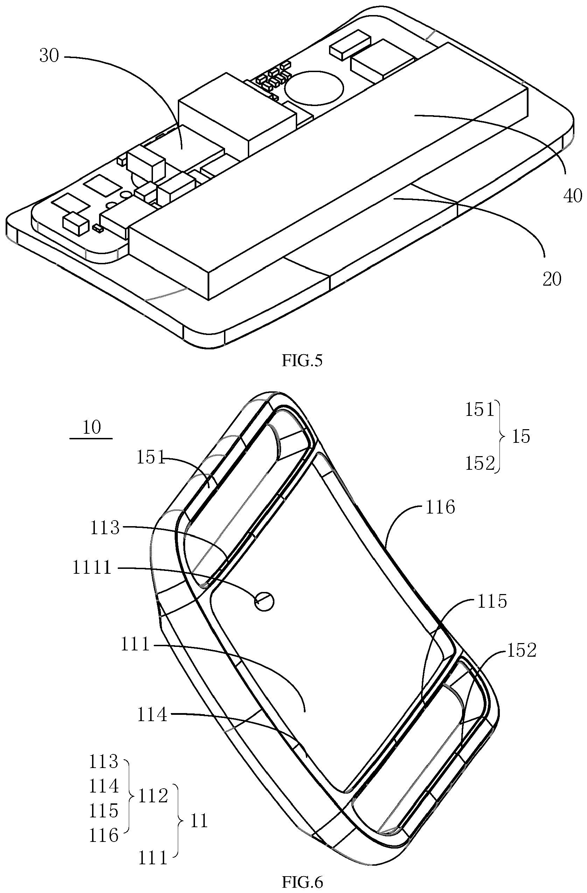

In some embodiments, as shown in FIG. 3, FIG. 4 and FIG. 5, FIG. 3 is a front view of the electronic device 300 according to the embodiment shown in FIG. 1 of the present disclosure, FIG. 4 is a rear view of the electronic device 300 according to the embodiment shown in FIG. 1 of the present disclosure, and FIG. 5 is a partial structural view of the electronic device 300 according to the embodiment shown in FIG. 1 of the present disclosure. The electronic device 300 may include a first housing 10, a second housing 20, a mainboard assembly 30, a battery 40, an indicator light 50, a balancing electrode 60 and a measuring electrode 70.

The first housing 10 and the second housing 20 are snapped together to form a shell (also referred to as a "housing") of the electronic device 300, and the shell may be used to protect and carry the electronic components (such as the mainboard assembly 30, the battery 40, the bluetooth module, etc.) inside the electronic device 300. Therefore, the shell may also be called a "protective housing". The shell may be configured to connect with the strap 600 to form the above-mentioned ring structure, so as to realize the wearing of the electronic device 300. For example, the first end 301 and the second end 302 of the electronic device 300 in FIG. 1 are defined on the shell. The shell has an accommodating space for accommodating electronic components such as the mainboard assembly 30, the battery 40 and the like. The mainboard assembly 30, the balance electrode 60 and the measuring electrode 70 form an ECG signal detection circuit. The mainboard assembly 30 is configured to record the electrical activity change data generated by the heart every cardiac cycle from the user's body surface. The mainboard assembly 30 may control the normal function of the ECG signal detection. The mainboard assembly 30 may also control the charging function of the battery 40. For example, during the charging operation, the mainboard assembly 30 is connected to a power source such as the commercial power to provide electric power for the battery 40. The battery 40 may provide electric power for the normal function of the electronic device 300. The indicator light 50 is used to reflect the working status of the electronic device 300 such as the detection status of the ECG signals, the charging status of the battery 40, the power status of the battery 40 and the like. The balancing electrode 60 and the measuring electrode 70 are configured to contact with the user's body, such as the wrist or the hand, so as to realize the detection of the ECG signals.

As shown in FIG. 3, FIG. 4 and FIG. 6, FIG. 6 is a schematic structural view of the first housing 10 according to the embodiment shown in FIG. 3 of the present disclosure. The first housing 10 is configured to be fastened with the second housing 20, be configured to be connected with the strap 600 to realize the wearing of the wearable device 100, and be used for the installing of the indicator light 50 and the balance electrode 60. The first housing 10 may include a main housing body 11 and a connecting ring 15. The main housing body 11 is used to be buckled and connected with the second housing 20 to form an accommodating space. The connecting ring 15 may be arranged at two opposite ends of the main housing body 11, and to be connected with the strap 600 to realize the wearing of the wearable device 100. In an embodiment, the main housing body 11 and the connecting ring 15 may be integrally structured.

The housing body 11 may include a top plate 111 and a side wall 112 extended from an edge of the top plate 111 to a side of the second housing 20. The top plate 111 and the side wall 112 cooperatively form a groove-like structure, and when fastened with the second housing 20, the accommodating space may be formed.

In an embodiment, a through hole 1111 may be defined in the top plate 111. The through hole 1111 may allow the indicator light 50 to be at least partially installed in the hole. In an embodiment, the through hole 1111 may also allow some circuits of the mainboard assembly 30 to pass through and be connected to the electronic components such as the indicator light 50 or a display module (not shown, the display module is used for the display of information). It may be seen that the display module may also be taken as a part of the electronic device 300, and the display module may be installed on a side of the top plate 111 away from the second housing 20.

In an embodiment, the top plate 111 may be a rounded rectangle, may be made of hard materials, and of course may also be made of transparent materials. In an embodiment, the transparent top plate 111 may facilitate that the installation position of the indicator light 50 may be moved from the through hole 1111 into the accommodating space, such that the indicator light 50 may perform information indication in the accommodating space. In this case, the opening of the through hole 1111 may also be avoided, thus guaranteeing the waterproofness and airtightness of the electronic device 300 on the other hand. In other words, the through hole 1111 may be omitted.

In an embodiment, the sidewall 112 may include a first sidewall 113, a second sidewall 114, a third sidewall 115, and a fourth sidewall 116 connected end to end in sequence. In an embodiment, the material of the side wall 112 may be the same as the material of the top plate 111.

In an embodiment, the structure of the connecting ring 15 is a ring structure, facilitating the wrapping of the strap 600. The number of the connecting rings 15 may be two, such as a first connecting ring 151 and a second connecting ring 152. Of course, the number of the connecting rings 15 may also be one, three, four, etc. The number of the connecting rings 15 is not specifically limited here.

In an embodiment, the first connecting ring 151 may be connected to one end of the strap 600, and the second connecting ring 152 may be connected to another end of the strap 600. In an embodiment, as illustrated in FIG. 6, the first connecting ring 151 is disposed on a side of the first side wall 113 away from the top plate 111. The second connecting ring 152 is disposed on a side of the third side wall 115 away from the top plate 111. In an embodiment, the first connecting ring 151 and the first side wall 113 are integrally structured. In an embodiment, the second connecting ring 152 and the third side wall 115 are integrally structured.

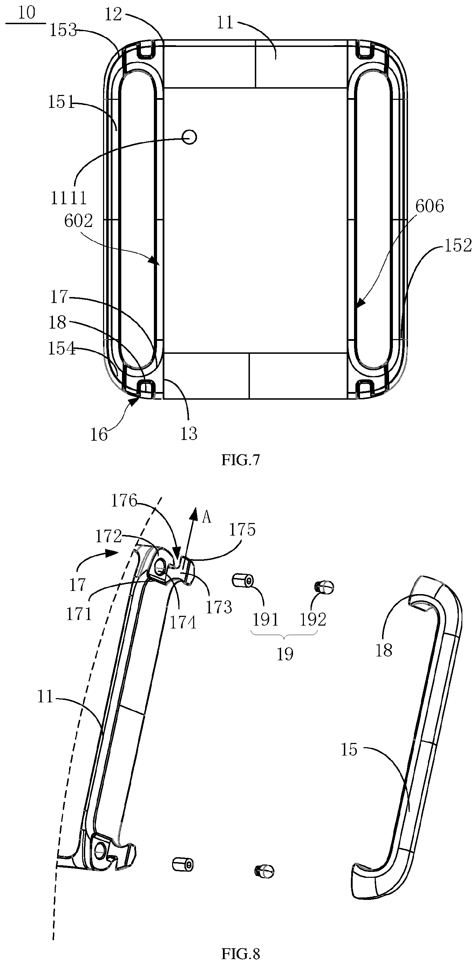

In an embodiment, as illustrated in FIG. 7, which is a schematic structural view of another embodiment of the first housing 10 according to the embodiment shown in FIG. 6 of the present disclosure. The first housing 10 is configured to be fastened with the second housing 20, is connected with the strap 600 to realize the wearing of the wearable device 100, and is used for the installing the indicator light 50 and the balance electrode 60. The first housing 10 may include a main housing body 11 and a connecting ring 15. The main housing body 11 is configured to be buckled and connected with the second housing 20 to form an accommodating space. The connecting ring 15 is arranged at two opposite ends of the main housing body 11 to be connected with the strap 600 so as to realize the wearing of the wearable device 100. The general structure of the main housing body 11 may be seen in FIG. 6, and will not be repeated here.

In an embodiment, the structure of the connecting ring 15 may be a columnar structure. A first end 153 and a second end 154 are provided in the connecting ring 15. A position at the first end 153 is bent toward one side of the main housing body 11 and a position at the second end 154 is bent toward one side of the main housing body 11, such that the first end 153 and the second end 154 are connected to the main housing body 11 to form a ring structure. The connecting ring 15 is used for binding the strap 600. The number of the connecting rings 15 may be two, such as a first connecting ring 151 and a second connecting ring 152. Of course, the number of the connecting rings 15 may also be one, three, four, etc. The number of the connecting rings 15 is not specifically limited here. In an embodiment, as shown in FIG. 1, the first connecting ring 151 may be connected to one end of the strap 600, and the second connecting ring 152 may be connected to another end of the strap 600.

In an embodiment, as shown in FIG. 7, a first connecting portion 12 and a second connecting portion 13 are provided on the main housing body 11, facilitating the connection with the connecting ring 15. In an embodiment, the connecting ring 15, such as the first end 153 of the first connecting ring 151, is configured to be connected with the first connecting portion 12 of the main housing body 11, and the connecting ring 15, such as the second end 154 of the first connecting ring 151, is configured to be connected with the second connecting portion 13 of the main housing body 11, such that the connecting ring 15 and the main housing body 11 may form a ring structure. In an embodiment, the structure of the connecting ring 15 such as the second connecting ring 152 may be consistent with the structure of the first connecting ring 151, so the specific structure of the second connecting ring 152 may be referred to the first connecting ring 151, which will not be repeated here. Of course, at least one of the first connecting ring 151 and the second connecting ring 152 in the embodiment shown in FIG. 7 may adopt the structure of the connecting ring 15 shown in FIG. 6.

It may be understood that the connecting ring 15 shown in FIG. 7 may also be a ring structure, and only one part is connected with the main housing body 11.

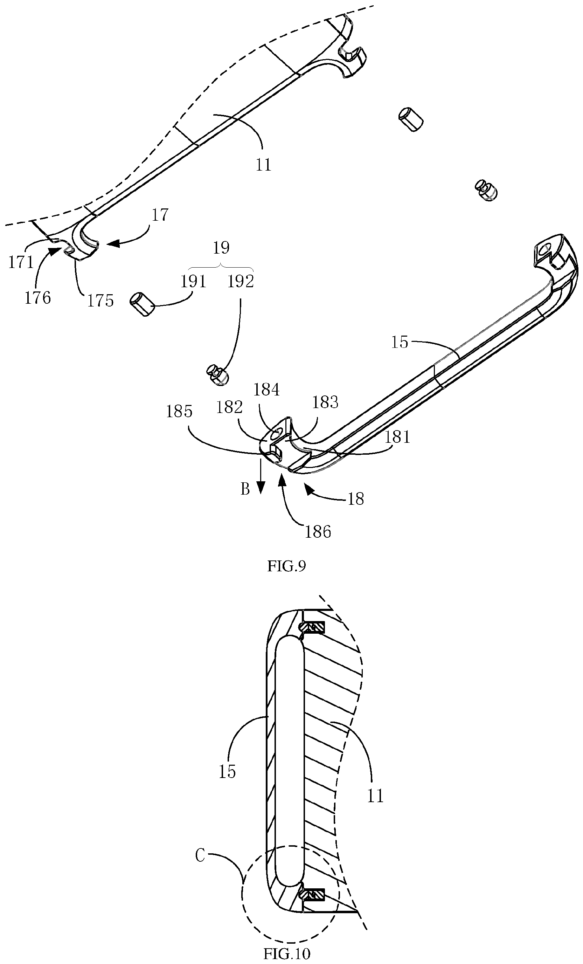

In an embodiment, as shown in FIG. 7, FIG. 8 and FIG. 9, FIG. 8 is an exploded schematic view of the detachable connector 16 according to the embodiment shown in FIG. 7, FIG. 9 is another exploded schematic view of the detachable connector 16 according to the embodiment shown in FIG. 7. In order to realize the detachable connection of the connecting ring 15 and the main housing body 11, the detachable connector 16 may be arranged at the junction position of the connecting ring 15 and the main housing body 11, such as the junction position of the first end 153 and the first connecting portion 12, the junction position of the second end 154 and the second connecting portion 13. Through the cooperation of the detachable connector 16, the connection and detachment of the connecting ring 15 and the main housing body 11 may be realized simply and conveniently. The detachable connector 16 may include a first connecting member 17, a second connecting member 18 and a locking member 19. The first connecting member 17 and the second connecting member 18 are connected or disconnected with the cooperation of the locking member 19.

In an embodiment, as shown in FIG. 7, the first connecting member 17 is disposed on the main housing body 11, and the second connecting member 18 is disposed on the connecting ring 15 (such as the first connecting ring 151). In an embodiment, the first connecting member 17 may be disposed on the connecting ring 15(such as the first connecting ring 151), and the second connecting member 18 may be disposed on the main housing body 11. Understandably, the first end 153 is connected to the first connecting portion 12 through the detachable connector 16, and the second end 154 is connected to the second connecting portion 13 through the detachable connector 16. Both of the first connecting ring 151 and the second connecting ring 152 may be arranged at the first end 153 or the second end 154.

In an embodiment, as shown in FIG. 8, the first connecting member 17 may include a first main body 171 and a first bump 175. The first main body 171 is used to be connected with the second connecting member 18 through the locking member 19, and the first bump 175 is used to be bucked with the second connecting member 18 in a staggered way.

The first main body 171 is provided with a first contact plane 172 and a second contact plane 173. The first contact plane 172 abuts against the second connecting member 18 and the second contact plane 173 abuts against the second connecting member 18 to realize the positioning of the first connecting member 17 and the second connecting member 18. In an embodiment, the first contact plane 172 and the second contact plane 173 may be perpendicular to each other such that the second connecting member 18 may slide relatively to the first connecting member 17 along the extension direction of the first contacting plane 172 of the first connecting member 17 to achieve connection and disconnection.

In an embodiment, as shown in FIG. 8, the first contact plane 172 is provided with a first accommodating groove 174 for accommodating the locking member 19. Therefore, it's convenient for the locking member 19 to connect the first connecting member 17 and the second connecting member 18, and the locking member 19 may be triggered conveniently when the second connecting member 18 slides in the extension direction of the first contact plane 172, facilitating the realization of the locked and non-sliding state of the first connecting member 17 and the second connecting member 18.

In an embodiment, the first bump 175 protrudes from an edge of the second contact plane 173. The first bump 175 and the first main body 171 cooperatively form a first socket 176. The first bump 175 cooperates with the first socket 176 to form a first buckling position, realizing a staggered fastening with the second connecting member 18.

In an embodiment, the first bump 175 protrudes along the first direction A at an edge of the second contact plane 173. The first direction A is parallel to the first contact plane 172 and the first direction A is parallel to the second contact plane 173. As shown in FIG. 8, two first connecting members 17 are provided on the main housing body 11, the first bump 175 of one of the first connecting members 17 protrudes away from the other first connecting member 17. As a result, a stable connection of the main housing body 11 and the connection ring 15 is realized. Of course, among the two first connecting members 17 provided on the main housing body 11, the first bump 175 of one of the first connecting members 17 may also protrude toward the other first connecting member 17.

In an embodiment, as shown in FIG. 9, the second connecting member 18 may include a second main body 181 and a second bump 185. The second main body 181 is configured to be connected with the first connecting member 17 through the locking member 19, and the second bump 185 is configured to be buckled with the first connecting member 17 in a staggered way.

The second main body 181 is provided with a third contact plane 182 and a fourth contact plane 183. The third contact plane 182 abuts against the first connecting member 17 and the fourth contact plane 183 abuts against the first connecting member 17 to realize the positioning of the first connecting member 17 and the second connecting member 18. In an embodiment, the third contact plane 182 is in contact with the first contact plane 172 and the fourth contact plane 183 is in contact with the second contact plane 173, such that the positioning of the first connecting member 17 and the second connecting member 18 is realized.

In an embodiment, as illustrated in FIG. 9, the third contact plane 182 is provided with a second accommodating groove 184 for accommodating the locking member 19, so as to facilitate the locking member 19 to connect the first connecting member 17 and the second connecting member 18. In an embodiment, when the first contact plane 172 of the first connecting member 17 is in contact with the third contact plane 182 of the second connecting member 18, the second connecting member 18 slides in the extension direction of the first contact plane 172 until the second contact plane 173 is in contact with the fourth contact plane 183, the first accommodation groove 174 is opposite to the second accommodation groove 184 and forms an integral space for accommodating the locking member 19.

In an embodiment, the third contact plane 182 is perpendicular to the fourth contact plane 183, such that the first connecting member 17 and the second connecting member 18 may relatively slide to each other to achieve the connection and disconnection of each other.

In an embodiment, the second bump 185 protrudes from an edge of the fourth contact plane 183. The second bump 185 and the second main body 181 form a second socket 186. The second bump 185 cooperates with the second socket 186 to form a second buckling position, such that the second bump 185 and the first connecting member 17 may be buckled together in a staggered way. In an embodiment, when the first connecting member 17 is engaged with the second connecting member 18, the first bump 175 is located in the second socket 186, and the second bump 185 is located in the first socket 176, realizing the staggered fastening of the first connecting member 17 and the second connecting member 18.

In an embodiment, the second bump 185 protrudes along the second direction B at the edge of the fourth contact plane 183. The second direction B is parallel to the third contact plane 182 and parallel to the fourth contact plane 183.

In an embodiment, as shown in FIG. 9, two second connecting members 18 are provided on the connecting ring 15, and the second bump 185 of one of the second connecting members 18 is located on the fourth contact plane 183 away from the edge of one side of the other second connecting member 18. Of course, for the two second connecting members 18 provided on the connecting ring 15, the second bump 185 of one of the second connecting members 18 may also be located at the fourth contact plane 183 close to the edge of one side of the other second connecting member 18. The two second bumps 185 extend towards the same direction.

As shown in FIG. 8 and FIG. 9, the locking member 19 may be placed in both of the first accommodating groove 174 and the second accommodating groove 184, thus avoiding the relative sliding of the first connecting member 17 and the second connecting member 18. Moreover, the staggered fastening of the first buckling position and the second buckling position may prevent the relative rotation of the first connecting member 17 and the second connecting member 18. At the same time, the first connecting member 17 may be prevented from moving to a side away from the second connecting member 18, and the second connecting member 18 may be prevented from moving to a side away from the first connecting member 17, completely realizing the connection between the first connecting member 17 and the second connecting member 18, and further realizing the connection between the connecting ring 15 and the main housing body 11.

In an embodiment, the locking member 19 may include an elastic member 191 and a locking head 192. The elastic member 191 and the locking head 192 are disposed in the first accommodating groove 174. One end of the elastic member 191 abuts against the first main body 171 in the elastic direction, and the other opposite end of the elastic member 191 abuts against the locking head 192, such that the locking head 192 may be at least partially placed in the first accommodating groove 174. When one side of the locking head 192 away from the elastic member 191 is not squeezed, the stretching force of the elastic member 191 pushes the locking head 192 to slide in a direction away from the side of the elastic member 191, and then the locking head 192 is partially placed in the first accommodating groove 174. When one side of the locking head 192 away from the elastic member 191 is squeezed, the locking head 192 is subjected to an extrusion force such that the locking head 192 slides in a direction close to the side of the elastic member 191, and then the whole locking head 192 is placed in inside the containing groove 174.

In an embodiment, the elastic member 191 may be a spring or be made of an elastic plastic material, and of course the elastic member 191 may also be made of other elastic materials.

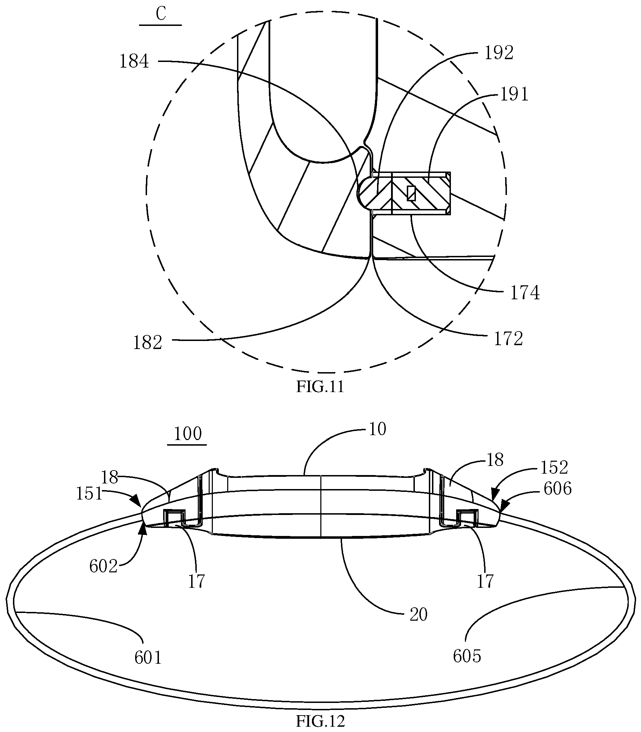

In an embodiment, as shown in FIG. 10 and FIG. 11, FIG. 10 is a partial cross-sectional view of the main housing body 11 and the connecting ring 15 connected to each other according to the embodiment shown in FIG. 9 of the present disclosure, FIG. 11 is a cross-sectional view of a part C of the main housing body 11 and the connecting ring 15 connected to each other according to the embodiment shown in FIG. 10 of the present disclosure. An end of the locking head 192 away from the elastic member 191 is a spherical surface, facilitating the compression of the locking head 192that follows the relative sliding to each other of the first connecting member 17 and the second connecting member, and realizing the connection and disconnection between the first connecting member 17 and the second connecting member 18. In an embodiment, in order to disassemble of the first connecting member 17 and the second connecting member 18, the length of the locking head 192 exposed to the first accommodating groove 174 is equal to the radius of the spherical end of the locking head 192.

In an embodiment, in order to prevent the locking head 192 from sliding out of the first accommodation groove 174 completely, for example, from sliding out of the mouth of the first accommodating groove 174, a limiting member may be provided in the first accommodating groove 174. For example, structures such as bumps, protruding rings, and screws may be used to limit the locking head 192 such that the locking member 19 is at least partially placed in the first accommodating groove 174.

It may be understood that the locking member 19 may also not be arranged in the first accommodating groove 174, but be arranged in the second accommodating groove 184 instead. The arrangement manner of the locking member 19 shown in FIG. 8 may be referred as the arrangement manner of the locking member 19 being disposed in the second accommodating groove 184.

When the connecting ring 15 is to be connected to the main housing body 11, the first contact plane 172 is in contact with the third contact plane 182, such that the first bump 175 is facing the second socket 186, and the second bump 185 is facing the first socket 176, then the connecting ring 15 slides in the extension direction of the first contact plane 172 until the second contact plane 173 contacts the fourth contact plane 183. During the sliding process, the first bump 175 gradually slides into the second socket 186, and the second bump 185 gradually slides into the first socket 176. In addition, during the sliding process, the locking head 192 is squeezed by the second connecting member 18 and completely slides into the first accommodating groove 174, and the elastic member 191 is squeezed and in a compressed state. When the second contact plane 173 is in contact with the fourth contact plane 183, the first accommodating groove 174 is opposite to the second accommodating groove 184, and under the stretching force of the elastic member 191, the locking head 192 is ejected and slides into the second accommodating groove 184. In this case, the locking head 192 is partially placed in the first accommodating groove 174, and partially placed in the second accommodating groove 184. The disassembling process may be operated in reverse.

Of course, in order to facilitate the installation and disassembly of the first connecting member 17 and the second connecting member 18, an adjustment hole the same as the first accommodating groove 174 may also be provided in the first main body 171 of the first connecting member 17. Through the adjustment hole, an instrument may be used to abut against the locking head 192 so a force is implemented on the locking head 192 and the force may push the locking head 192 to slide toward one side of the elastic member 191, realizing the installation and disassembly of the first connecting member 17 and the second connecting member 18.

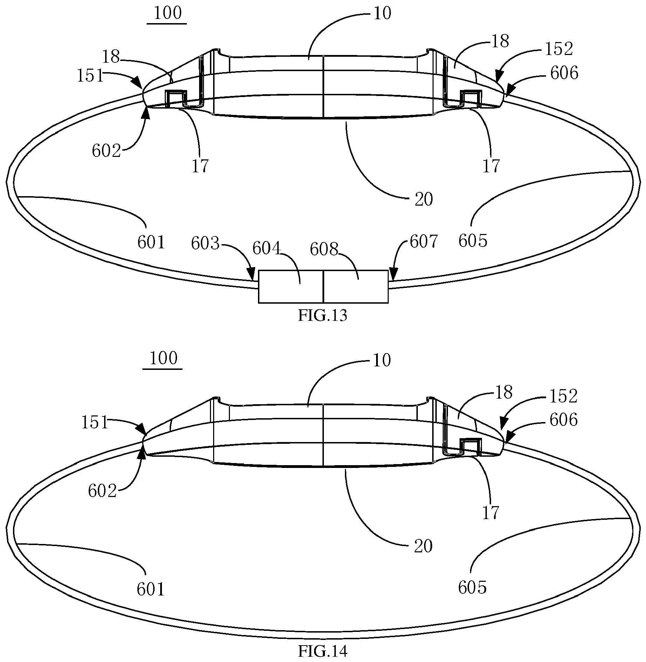

As shown in FIG. 12 and FIG. 13, FIG. 12 is a schematic structural view of the electronic device 300 when worn by the user according to the embodiment shown in FIG. 7 of the present disclosure, FIG. 13 is another schematic structural view of the electronic device 300 when worn by the user according to the embodiment shown in FIG. 7 of the present disclosure. Both of the first connecting ring 151 and the second connecting ring 152 of the first housing 10 are connected to the main housing body 11 through the detachable connecting member 16. One end of the strap 600 is connected to the first connecting ring 151, and one end of the strap 600 is connected to one end of the second connecting ring 152.

As shown in FIG. 14, which is a schematic structural view of another embodiment of the electronic device 300 when worn by the user according to the embodiment shown in FIG. 12 of the present disclosure. The first connecting ring 151 of the first housing 10 and the main housing body 11 are integrally structured, and the main housing body 11 and the second connecting ring 152 are connected by a detachable connecting member 16. One end of the strap 600 is connected to the first connecting ring 151, and one end of the strap 600 is connected to one end of the second connecting ring 152. Of course, at least one of the first connecting ring 151 and the second connecting ring 152 may be integrated with the main housing body 11.

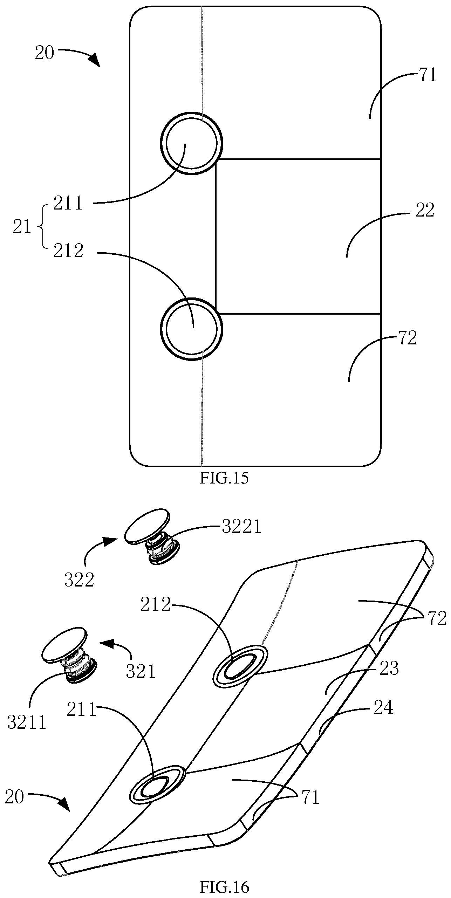

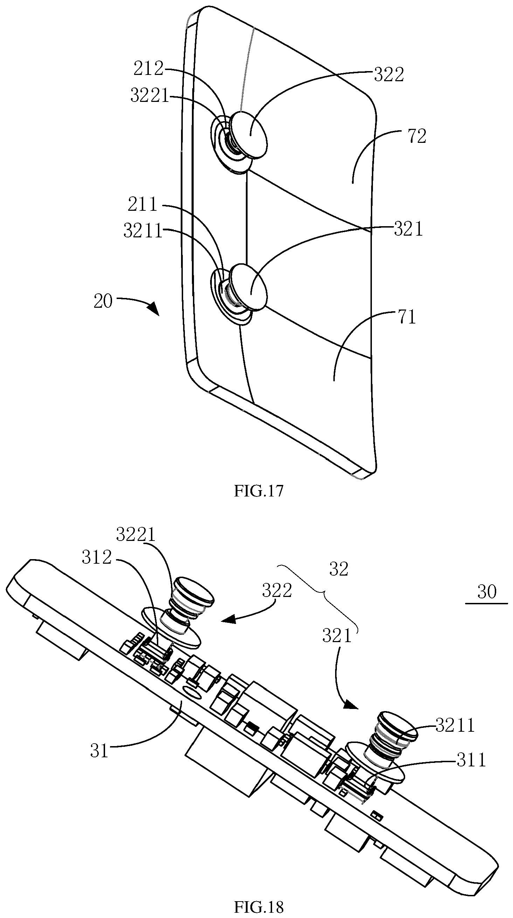

As shown in FIG. 4, FIG. 15, FIG. 16, and FIG. 17, FIG. 15 is a schematic structural view of the second housing 20 according to the embodiment shown in FIG. 4 of the present disclosure, FIG. 16 is a partial schematic structural view of the electronic device 300 according to the embodiment shown in FIG. 4 of the present disclosure, FIG. 17 is a partial structural schematic view of the electronic device 300 according to the embodiment shown in FIG. 4 of the present disclosure. The second housing 20 may be buckled with the first housing 10, and may be provided with the measuring electrode 70 and some parts of the mainboard assembly 30. The second housing 20 may be a plate-like structure. The second housing 20 may be made of rigid insulating material, so the second housing 20 may also be called "insulating housing". In an embodiment, the second housing 20 may be made through plastic injection molding process. The surface of the second housing 20 facing the top plate 111 and the surface away from the top plate 111 may be planar. Of course, the surface of one side of the second housing 20 facing the top plate 111 may be convex to the side close to the top plate 111 to form a curved surface, or may be concave to the side away from the top plate 111 to form a curved surface. The surface of one side of the second housing 20 away from the top plate 111 may be concave to the side close to the top plate 111 to form a curved surface, or may be convex to the side away from the top plate 111 to form a curved surface.

In an embodiment, mounting holes 21 such as a first mounting hole 211 and a second mounting hole 212 may be defined in the second housing 20 for mounting some parts of the mainboard assembly 30.

In an embodiment, the surface of the second housing 20 away from the top plate 111 is a contact surface 22 for being contacted with the human body. The measuring electrode 70 may be set on the contact surface 22 to facilitate the contact between the measuring electrode 70 and the human body, and then the human body, the measuring electrode 70 and the mainboard assembly 30 form an ECG signal measurement circuit.

In an embodiment, the second housing 20 may be fixed with the first housing 10 through buckle structure, screw connection structure, adhesive bonding, etc. The second housing 20 is connected to one side of the side wall 112 (such as the first side wall 113, the second side wall 114, the third side wall 115, and the fourth side wall 116) away from the top plate 111. The edge of the surface of the second housing 20 away from the top plate 111 is flush with the edge of the side wall 112 away from the top plate 111.

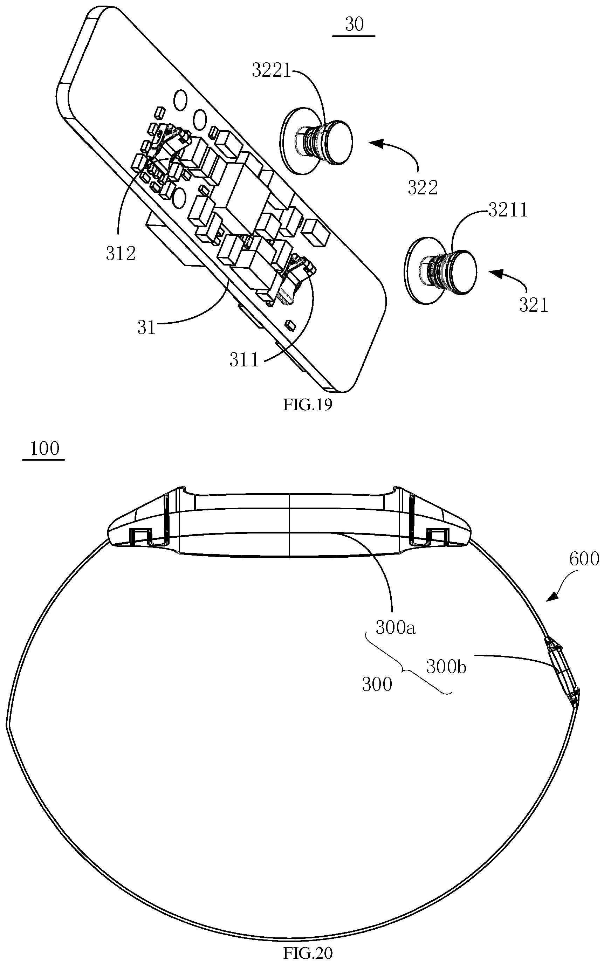

As shown in FIG. 18 and FIG. 19, FIG. 18 is a schematic structural view of the mainboard assembly 30 according to the embodiment shown in FIG. 5 of the present disclosure, and FIG. 19 is an exploded schematic view of the mainboard assembly 30 according to the embodiment shown in FIG. 18. The mainboard assembly 30 is installed in the accommodating space, and is configured to be electrically connected with the battery 40, the indicator light 50, the balance electrode 60, the measuring electrode 70, and the display module. Therefore, it is convenient for the mainboard assembly 30 to control the battery 40 to perform charging and discharging operations, for the mainboard assembly 30 to control the indicator light 50 to present the working state of the wearable device 100, for the mainboard assembly 30 to complete the ECG signal detection through the balance electrode 60 and the measuring electrode 70, for the mainboard assembly 30 to control the display module to display information such as an electrocardiogram generated by the ECG signals, and for the mainboard assembly 30 to control the wearable device 100 to exchange data with external devices. The mainboard assembly 30 may include a mainboard 31 and a first connecting head 32. Various electronic components such as processors and sensors may be integrated on the mainboard 31. The mainboard 31 may be fixed on the first housing 10 by screws, glue and other structures. The mainboard 31 may be electrically connected to the first connecting head 32, the battery 40, the indicator light 50, the balance electrode 60, the measuring electrode 70, and the display module, such that power transmission may be realized. For example, the first connecting head 32 may be connected to the commercial power to charge the battery 40. For example, the mainboard 31 may control the battery 40 to supply power to the indicator light 50, the balance electrode 60, the measuring electrode 70, and the display module. The mainboard 31 may be provided with a driven-right-leg circuit so as to be electrically connected with the balanced electrode 60. The driven-right-leg circuit is essentially negative feedback. The function of the driven-right-leg circuit is to remove the common-mode signal of the input amplifier by adopting the common-mode voltage as the reverse input of the drive circuit, so as to improve the common mode rejection ratio (CMRR) and realize the accuracy of the ECG parameters obtained from the ECG signals collected by the measuring electrode 70.

In an embodiment, elastic members such as a first elastic member 311 and a second elastic member 312 are disposed on the mainboard 31, such that the elastic members such as the first elastic member 311 and the second elastic member 312 abut against the first connecting head 32 to realize electrical connection.

In an embodiment, the first connecting head 32 may be a pogo pin connector. The first connecting head 32 may include a first conductive column 321 and a second conductive column 322. The mainboard 31 may be connected to a power source such as the commercial power to obtain electric energy through the first connecting head 32 such as the first conductive column 321 and the second conductive column 322. The mainboard 31 may be electrically connected with the measuring electrode 70 through the first connecting head 32 (such as the first conductive column 321 and the second conductive column 322), such that the measuring electrode 70 may be connected with the mainboard 31 to form an ECG signal detection circuit. In an embodiment, the processor on the mainboard 31 calculates the user's heart rate after processing the ECG signals, and generates an electrocardiogram. A Bluetooth module may also be integrated on the mainboard 31, and the bluetooth transmission of the electrocardiogram may be realized through the Bluetooth module and an external device such as a mobile phone.

As shown in FIG. 17, the first connecting head 32 such as the first conductive column 321 and the second conductive column 322 are installed on the second housing 20 such as in the installation hole 21. Specifically, the first conductive column 321 is provided with a sealing ring 3211. The second conductive column 322 is provided with a sealing ring 3221. The first conductive column 321 is installed in the first installation hole 211, the second conductive column 322 is installed in the second installation hole 212, and the seal connection between the first connecting head 32 and the second housing 20 is realized through the sealing ring 3211 and the sealing ring 3221. The sealing ring 3211 and the sealing ring 3221 are elastic soft rubber rings. In an embodiment, both of the first conductive column 321 and the second conductive column 322 are interference-fitted with the second housing 20 to realize installation. In an embodiment, one end portion of the first connecting head 32 such as the first conductive column 321 and the second conductive column 322 away from the mainboard 31 is electrically connected to the measuring electrode 70. In an embodiment, one end portion of the first connecting head 32 such as the first conductive column 321 and the second conductive column 322 away from the mainboard 31 is configured to be connected to a power source such as the commercial power, such that the wearable device 100 may obtain electric energy.

As shown in FIG. 18, one end portion of one side of the first connecting head 32 such as the first conductive column 321 near the mainboard 31 abuts against the first elastic member 311 to realize electrical connection. One end portion of one side of the first connecting head 32 such as the second conductive column 322 away from the mainboard 31 abuts against the second elastic member 312 to realize electrical connection.

As shown in FIG. 3 and FIG. 4, the balance electrode 60 is electrically connected to the mainboard 31, and specifically may be connected to the driven-right-leg circuit on the mainboard 31. Therefore, the balance electrode 60 is also called "Driven-Right-Leg Electrode". The balance electrode 60 is disposed on one side of the first housing 10 away from the second housing 20. Specifically, the balance electrode 60 may be disposed on any position (such as the connection ring 15 and the top plate 111) of the first housing 10 that may be touched by the user's fingers and other parts. It is certain that in some embodiments, in order to install the display module, the balance electrode 60 may be disposed on the edge of the top plate 111 to reserve an installation position for the display module. The balance electrode 60 may cooperate with the measuring electrode 70 to form a complicated ECG signal measuring circuit. For example, the wearable device 100 may be worn on the left wrist of the user, and the measuring electrode 70 is in contact with the left wrist to measure the ECG signals, the balance electrode 60 is touched by the right hand, and the reverse common-mode ECG signals are generated after the mainboard 31 inverts the common mode ECG signals collected by the measuring electrode 70, then the reverse common-mode ECG signals are input to the human body through the balanced electrode 60 until the measurement is completed. The accuracy of ECG parameters may be improved. In an embodiment, the balance electrode 60 may be omitted.

As shown in FIG. 4 and FIG. 15, the measuring electrode 70 may be metal-plated, which is convenient to be contacted with the human body for ECG signal measurement and the transmission of ECG signals. A wear-resistant metal coating may be deposited on the surface of the second housing 20 by water plating and/or vacuum plating to obtain the measuring electrode 70. The measuring electrode 70 may also be a metal sheet arranged on the surface of the second housing 20 by means of bonding or bayonet connection. The measuring electrode 70 may include a first electrode 71 and a second electrode 72 arranged at intervals on the contact surface 22. In order to realize the construction of the ECG signal detection circuit, the first electrode 71 is electrically connected to the first conductive column 321, and the second electrode 72 is electrically connected to the second conductive column 322, such that the first electrode 71, the first conductive column 321, the second conductive column 322, and the mainboard 31 are connected to form an ECG signal detection circuit, and the mainboard 31 may obtain ECG parameters according to the ECG signals collected by the first electrode 71 and the second electrode 72.

As shown in FIG. 16, one surface of one side of the second housing 20 near the top plate 111 is a first surface 23, and one surface (i.e., the side surface) connecting the first surface 23 and the contact surface 22 is a connecting surface 24. In addition to being arranged on the contact surface 22, the first electrode 71 and the second electrode 72 may also be arranged on the first surface 23 or the connecting surface 24. As a result, the first electrode 71 is electrically connected to the first conductive column 321 in the accommodating space, and the second electrode 72 is electrically connected to the second conductive column 322 in the accommodating space.

It may be understood that a portion of the first electrode 71 located on the contact surface 22 may be directly electrically connected to the first conductive column 321, and a portion of the second electrode 72 located on the contact surface 22 may be directly electrically connected to the second conductive column 322.

In an embodiment, as shown in FIG. 1, the strap 600 is configured to be connected with the human body to realize the wearing of the wearable device 100. The strap 600 may include a first strap 601 and a second strap 605. A first end 602 of the first strap 601 is connected to the first end 301 (in other words, the connection ring 15) of the electronic device 300, and a first end 606 of the second strap 605 is connected to the second end 302 (in other words, the connection ring 15) of the electronic device 300. The wearing of the wearable device 100 may be realized by the ring structure formed by connecting the first strap 601, the second strap 605, and the electronic device 300 sequentially from end to end.

In an embodiment, as shown in FIG. 2, an integral structure is formed due to that the first end 602 of the first strap 601 is connected to the first end 606 of the second strap 605. The electronic device 300 is fixed on the strap 600. The second end 603 of the first strap 601 is connected to the second end 607 of the second strap 605 through a third connecting member 604 and a fourth connecting member 608. In an embodiment, the third connecting member 604 and the fourth connecting member 608 are bayonet-connected. In an embodiment, one of the third connecting member 604 and the fourth connecting member 608 may be one of the first connecting member 17 and the second connecting member 18, and the other one of the third connecting member 604 and the fourth connecting member 608 may be the other one of the first connecting member 17 and the second connecting member 18.

In an embodiment, as shown in FIG. 12 and FIG. 14, the second end 603 of the first strap 601 is connected with the second end 607 of the second strap 605 to form an integral structure. The first end 602 of the first strap 601 is connected to the first end 301 (in other words, the first connection ring 151) of the electronic device 300, and the first end 606 of the second strap 605 is connected to the second end 302 (in other words, the first connection ring 151) of the electronic device 300. The wearing of the wearable device 100 is realized by the ring structure formed through connecting the first strap 601, the second strap 605, and the electronic device 300 sequentially from end to end.

In an embodiment, as shown in FIG. 13, the first end 602 of the first strap 601 is connected to the first end 301 (in other words, the first connection ring 151) of the electronic device 300, and the first end 606 of the second strap 605 is connected to the second end 302 (in other words, the second connection ring 152) of the electronic device 300. The second end 603 of the first strap 601 is connected to the second end 607 of the second strap 605 through the third connecting member 604 and the fourth connecting member 608. In an embodiment, the third connecting member 604 and the fourth connecting member 608 are bayonet-connected. In an embodiment, one of the third connecting member 604 and the fourth connecting member 608 may be one of the first connecting member 17 and the second connecting member 18, and the other one of the third connecting member 604 and the fourth connecting member 608 may be the other one of the first connecting member 17 and the second connecting member 18.

As shown in FIG. 20, which is a schematic structural view of a wearable device 100 according to another embodiment of the present disclosure. The wearable device 100 may include an electronic device 300 and a strap 600. In the wearable device 100, the electronic device 300 and the strap 600 may be disposed in the way described in the above-mentioned embodiments, and the electronic device 300 may be worn on the user's body such as the wrist and the head through the strap 600.

Specifically, the number of the electronic devices 300 may be two, such as an electronic device 300a and an electronic device 300b. Each of the electronic device 300a and the electronic device 300b may be replaced by the electronic device 300 shown in FIG. 3.

In an embodiment, the electronic device 300a may not be provided with the balancing electrode 60 and the measuring electrode 70, and the Bluetooth module may be provided in the mainboard assembly 30 of the electronic device 300a, and the electronic device 300a may be provided with the display module. The electronic device 300b may not be provided with the display module, and the Bluetooth module may be provided in the mainboard assembly 30 of the electronic device 300b. The Bluetooth module in the electronic device 300b may be paired with the Bluetooth module in the electronic device 300a for communication connection. Of course, in some embodiments, the communication connection may not be performed through the Bluetooth module, but may be directly connected by wires.

When in use, the ECG parameters may be measured through the balance electrode 60 and the measuring electrode 70 of the electronic device 300b. Under the control of the mainboard 31 in the electronic device 300b, the Bluetooth module of the electronic device 300 sends the ECG parameters to the electronic device 300a, and the Bluetooth module in the device 300a receives the ECG parameters. The ECG parameters may be displayed on the display module in the electronic device 300a under the control of the mainboard 31 in the electronic device 300a.

The above is only the embodiments of the present disclosure, and does not limit the patent scope of the present disclosure. Any equivalent structure or equivalent process conversion made by using the specification and drawings of the present disclosure, or directly or indirectly used in other related technologies fields, are all included in the scope of patent protection of this present disclosure in the same way.

## Claims

1. An electronic device, **characterized by** comprising:
a housing, having a contact surface on an outer surface thereof and an accommodation space inside;
a first connecting head, arranged on the housing, and configured to be electrically connected with a power source to transmit electric energy to the electronic device;
a first electrode and a second electrode, arranged on the contact surface apart from each other, and configured as measuring electrodes of an electrocardiogram (ECG) signal detection circuit, wherein each of the first electrode and the second electrode is electrically connected to the first connecting head; and
a mainboard, electrically connected to the first connecting head, and configured to obtain ECG parameters according to ECG signals collected by the first electrode and the second electrode.

2. The electronic device as claimed in claim 1, wherein the housing comprises:
a first housing; and
a second housing, connected with the first housing to define an accommodating space; wherein the contact surface is arranged on one side of the second housing away from the first housing, and the first connecting head is arranged on the second housing.

3. The electronic device as claimed in claim 2, wherein the first connecting head is a pogo pin connector, the pogo pin connector comprises a first conductive column and a second conductive column, the first electrode is electrically connected to the first conductive column, and the second electrode is electrically connected to the second conductive column.

4. The electronic device as claimed in claim 3, wherein the second housing is provided with a mounting hole, the first conductive column and the second conductive column are placed in the mounting hole and are sealed and connected to the second housing.

5. The electronic device as claimed in claim 3, wherein the second housing is provided with a first surface facing the first housing, the second housing is provided with a connecting surface connecting the contact surface and the first surface, and the first electrode and the second electrode are extended on the first surface through the connecting surface.

6. The electronic device as claimed in claim 5, wherein a part of the first electrode located on the first surface is electrically connected to the first conductive column, and a part of the second electrode located on the first surface is electrically connected with the second conductive column.

7. The electronic device as claimed in claim 3, wherein a part of the first electrode located on the contact surface is electrically connected to the first conductive column, and a part of the second electrode located on the contact surface is electrically connected to the second conductive column.

8. The electronic device as claimed in claim 3, wherein the mainboard is provided with a first elastic member and a second elastic member, the first elastic member is elastically abutted against the first conductive column such that the first conductive column is electrically connected to the mainboard, and the second elastic member is elastically abutted against the second conductive column such that the second conductive column is electrically connected to the mainboard.

9. The electronic device as claimed in claim 2, wherein a balance electrode is provided on the first housing, the balance electrode is electrically connected to the mainboard, and the balance electrode is configured to output reverse common-mode ECG signals inverted by the mainboard through processing common mode ECG signals collected by the first electrode and the second electrode.

10. A wearable device, **characterized by** comprising:
a housing, having a contact surface on an outer surface thereof and an accommodating space inside;
a first connecting head, arranged on the housing, and configured to be electrically connected to a power source to transmit electric energy;
a first electrode and a second electrode, arranged on the contact surface apart from each other, and configured as measuring electrodes of the electrocardiogram (ECG) signal detection circuit; wherein each of the first electrode and the second electrode is electrically connected to the first connecting head;
a mainboard, electrically connected to the first connecting head, and configured to obtain ECG parameters according to ECG signals collected by the first electrode and the second electrode; and
a strap, connected to the housing so as to be worn by the user.

11. The wearable device as claimed in claim 10, wherein a first connecting member is provided at a first end of the housing, and a second connecting member is provided at a first end of the strap.

12. The wearable device as claimed in claim 10, wherein the housing is arranged on the strap, the strap is provided with a first connecting member and a second connecting member capable of being connected to each other, the first connecting member is arranged at a second end of the strap, and the second connecting member is arranged at a first end of the strap.

13. The wearable device as claimed in claim 11 or 12, wherein the first connecting member is connected to the second connecting member, and the first connecting member is provided with a first contact plane and a second contact plane; the first contact plane is provided with a first accommodating groove, the second contact plane is provided with a first buckling position; the second connecting member is provided with a third contact plane and a fourth contact plane; and the third contact plane is provided with a second accommodating groove, and the fourth contact plane is provided with a second buckling position;
wherein, when the first connecting member is connected to the second connecting member, the first contact plane is in contact with the third contact plane, the second contact plane is in contact with the fourth contact plane, the first buckling position is buckled with the second buckling position in a staggered way, and the first accommodating groove is opposite to the second accommodating groove; a locking member is arranged between the first connecting member and the second connecting member, and the locking member is arranged in the first accommodating groove, and a part of the locking member is placed in the second accommodating groove to connect the first connecting member and the second connecting member.

14. The wearable device as claimed in claim 13, wherein the first contact plane is perpendicular to the second contact plane, and the third contact plane is perpendicular to the fourth contact plane.

15. The wearable device as claimed in claim 13, wherein the first connecting member comprises:
a first main body, provided with the first contact plane and the second contact plane;
a first bump, protruded from an edge of the second contact plane, and forms a first socket with the first main body, and the first socket and the first bump cooperatively define the first buckling position.

16. The wearable device as claimed in claim 15, wherein the second connecting member comprises:
a second main body, provided with the third contact plane and the fourth contact plane;
a second bump, arranged at an edge of the fourth contact plane, the second bump and the second body cooperatively form a second socket, and the second socket and the second bump define the second buckling position;
wherein, when the first buckling position and the second buckling position are fastened in a staggered way, the first bump is located at the second socket, and the second bump is located at the first socket.

17. The wearable device as claimed in claim 16, wherein the first bump protrudes along a first direction at the edge of the second contact plane, and the second bump protrudes along a second direction at the edge of the fourth contact plane; the first direction is parallel to the first contact plane and is parallel to the second contact plane, the second direction is parallel to the third contact plane and is perpendicular to the fourth contact plane.

18. The wearable device as claimed in claim 13, wherein the locking member comprises:
an elastic member, placed in the first accommodating groove;
a locking head, at least partially placed in the first accommodating groove; wherein one end of the elastic member abuts against the first connecting member, and an opposite end of the elastic member abuts against the locking head, and the locking head is configured to slide in the first accommodating groove during a compressing or stretching process of the elastic member.

19. The wearable device as claimed in claim 18, wherein the first connecting member is provided with a limiting member at a mouth of the first accommodating groove to prevent the locking head from sliding out from the mouth of the first accommodating groove.

20. The wearable device as claimed in claim 18, wherein the first connecting member and the second connecting member are configured as: the first buckling position and the second buckling position are buckled together in a staggered way and the locking head is partially placed in the second accommodating groove when the first contact plane is in contact with the third contact, and the first connecting member and the second connecting member slide relatively along the extension direction of the first contact plane until the second contact plane contacts the fourth contact plane.
